Europäisches Patentamt

European Patent Office

Office européen des brevets

(11) Publication number: **0 073 856**
**A1**

## (12) EUROPEAN PATENT APPLICATION

(21) Application number: **81200960.3**

(22) Date of filing: **28.08.81**

(51) Int. Cl.³: **C 12 N 7/00**
**A 61 K 39/215, A 61 K 39/295**

(43) Date of publication of application:
**16.03.83 Bulletin 83/11**

(84) Designated Contracting States:
**NL**

(71) Applicant: **Gist - Brocades N.V.**
**Wateringseweg 1 P.O. Box 1**
**NL-2600 MA Delft(NL)**

(72) Inventor: **Apontoweil, Peter**
**Burg. Martenslaan 39**
**NL-3956 EK Leersum(NL)**

(72) Inventor: **Krasselt, Manfred Max**
**Park Arenberg 59**
**NL-3731 EP De Bilt(NL)**

(74) Representative: **Van der Straaten, Jan Anthony et al,**
**c/o GIST-BROCADES N.V. Patents and Trademarks**
**Department Wateringseweg 1 P.O. Box 1**
**NL-2600 MA Delft(NL)**

(54) **Infectious-bronchitis vaccines for poultry, combined infectious-bronchitis vaccines, process for preparing such vaccines, process for preventing infectious bronchitis and infectious-bronchitis virus strain.**

(57) Infectious bronchitis vaccines for poultry derived from at least a virus strain of a novel serotype of the infectious bronchitis virus (IBV), identified by the internal indication Gelderland. 901, which has been deposited at the Czechoslovak National Collection of Type Cultures of the Institute of Hygiene and Epidemiology under the number CNCTC AO 17/81 and at the Collection Nationale de Cultures de Microorganismes d'Institut Pasteur, under no. I-168; combined infectious-bronchitis vaccines, derived from the IBV H.120 or the IBV H.52 of the Massachusetts type strain in addition to a novel before-mentioned virus strain; the infectious-bronchitis virus strain itself; and a process for preventing of infectious-bronchitis with poultry by vaccination with vaccines, derived from the before-mentioned strains.

Preferably live infectious bronchitis vaccines are prepared, which are containing a virus content of at least $10^{4.0}$ EID$_{50}$ pro dosage of each of the virus strains after freeze drying.

Croydon Printing Company Ltd.

Gist-Brocades N.V.


Infectious-bronchitis vaccines for poultry, combined infectious-bronchitis vaccines, process for preparing such
vaccines, process for preventing infectious bronchitis and
infectious-bronchitis virus strain.


The invention is relating to infectious bronchitis (IB)
vaccines for poultry, combined infectious-bronchitis vaccines
for poultry, a process for the preparation of such vaccines
and more particularly to improved infectious bronchitis
vaccines, derived from at least one infectious bronchitis
virus strain of a novel serotype as to those of the viruses,
which are known up to now for vaccination purposes.

The application of live infectious bronchitis
vaccines for poultry is already known for many years.

Infectious bronchitis is an important affection of
the respiratory system, the kidneys and oviduct of poultry.
The cause of this syndrome is a corona virus. The poultry is
severely affected by epizootics of this disease.

The infectious bronchitis causes a high mortality,
especially in young poultry. Besides mortality and more or
less strong respiratory symptoms, egg production drops occur,
as lesions to the oviduct and/or as a result of the stress
situation in which the poultry falls after an infection with
IB virus.

Moreover infections with IB virus may stimulate
latent virus- or bacterial infections and may give rise in
this way to severe economical losses, especially in the
broiler field.

For the combatment of infectious bronchitis as well
vaccines derived from inactivated virus as those ones derived
from live virus, are applied. However, it was found that in
some cases a loss of immunogenic properties occurred after
inactivation of these viruses with e.g. formaline and ultra
violet light (M.S. Hofstad, Diseases of Poultry, Biester and
Schwarte, Iowa University, Press. Ames. (1965), 615).

As sound chickens can be killed or diseased by primary vaccination with live, non or slightly atte-nuated virus vaccines, whereby an especial danger is existing for animals of less than 2 or 3 weeks old or for chickens shortly before the start or during laying, people skilled in this art have a clear preference for the application of dead vaccines which still have sufficient immunogenic properties, or of live vaccines, whereby was tried to increase the harmlessnes of such vaccines by means of attenuation of the original IB field virus isolates.

For such modified live virus vaccines, viruses having undergone 25 or more embryo passages to reduce their pathogenicity and their disseminating ability, are applied up to now, such as viruses derived from the Massachusetts type and more particularly the IBV W 48, M 41 and 82828 strains of this type, besides the Connecticut isolates, e.g. the A 5968 strain. The immunizing capacity of these viruses is very specific against either Massachusetts or Connecticut types of IB viruses. This in contrast to the IBV H52 and H120 strains, which have been passaged approximately 52 and 120 times respectively in embryonated chicken eggs, having a relatively broad immunizing capacity. The H-strain is presently applied on world wide scale on account of his broad immunisation spectre against among others Massachusetts and Connecticut types of IB-virus and has been isolated and attenuated by Bijlenga, Hoekstra and Ripsens, as is disclosed in Tijdschr. Diergeneesk. 81:43, "Infectious bronchitis in chicks in the Netherlands" (1956), Tijdschr. Diergeneesk. 85:320 (1960), Tijdschr. Diergeneesk. 85:279 (1960) and Tijdschr. Diergeneesk. 85:398 (1960).

Although the use of most vaccines of these modified strains has appeared to be fairly safe and effective, up to now, these vaccines appeare to be more and more unable to prevent outbreaks of infectious bronchitis in a sufficient way under certain conditions, as appears from Avian Diseases vol. 20, no. 1, p. 42 and 177 and Avian Diseases vol. 19, no. 2, p. 323 and 583. This shortcoming of the present IB vaccines is attributed to occurring antigenic variations of the virus in an important degree, as appears e.g. from Archiv

für die Gesamte Virusforschung 34, p. 32 (1971) and Cunningham C.H. Develop. Biol. Standard, 33, 311 (1976).

Efforts were made therefore to reach an adequate vaccination of poultry by means of preparation and application of combined vaccines, derived IBV strains of different serotypes, corresponding with the IBV types.

However, hereby a clearly encountered difficulty appeared to form the decrease of immunogenic properties of the respective starting viruses, caused by mutual interaction, as appears from Am. J. Vet. Res. 36, 4, 524 and 525 (1965) and Avian Diseases 12, 577 (1968).

The most adequate improvement which has been reached up to now against the nowadays frequently occuring IB virus infections caused by viruses deviating from the ones which can be combatted with vaccines derived from the H-strain, was obtained by the preparation and application of combined vaccines, derived from one or more of the IB viruses, identified by Utrecht.101, Utrecht.102, Drente.201, Limburg.501, Limburg.502, Brabant.801, Limburg.536, Overijssel.728 and Utrecht.121, as disclosed in the European patent application 0 030 063. However, there is a still existing need for further improved IB vaccines with immunizing properties over a wider range and/or better immunogenic properties.

It will be appreciated that the pursued improvement of these vaccines is still severely hampered, caused by the appearance of new serotypes of IB-viruses, the change of immunogenic and other properties of the presently available IB viruses after a large number of passages in embryonated chicken eggs and the lack of sufficiently effectively applicable serological and immunological test procedures. In this connection reference may be made to Avian Diseases, 19, 2, 323 and 324 (1975).

As a result of extensive research and experimentation, a novel IB virus could surprisingly be obtained and determined, which deviates from the frequently applied IB viruses of the H type (e.g. IB H120 and IB H52), but shows corresponding antigenic properties with the viruses described in the before-mentioned European patent application.

The frequently applied IB viruses of the H-type deviates from new IB virus in cross neutralization tests (virus neutralization tests) according to e.g. the method as described in American Association of Avian Pathologists, "Isolation and Identification of Avian Pathogens", page 184 (1975), in the understanding that antisera diluted in a ratio of 1:5 are applied, and in challenge experiments with subsequent virus reisolation tests. In other words at an inoculation with a virus of the H-type (e.g. IB H120 and IB H52), the concerning animals are not protected against virus replication in the mucosa of the respiratory system after a challenge with the beforementioned deviating novel IB virus. Antibodies against the IB H strain equally appeared not to be able to neutralize significant amounts of IB virus of the novel deviating type.

Of special importance for the practice is that the novel IB virus causes respiratory symptoms with animals showing high antibody titers against the IB H strain, and with still laying animals, egg production drops.

The new IB virus generates after inoculation antibodies against not only itself, but also against the IB viruses different from the H-type strains, as mentioned in the above-cited European patent application. This new IB virus therefore shows a broad spectrum against the nowadays frequently occuring IB virus strains deviating from the ones which can be combatted with vaccines derived from the H-strain.

The novel virus, which forms a feature of the present invention is identified by the internal notation Gelderland.901 (G.901), deposited at the Czechoslovak National Collection of Type Cultures of the Institute of Hygiene and Epidemiology in Prague under no. CNCTC AO 17/81 and at the Collection Nationale de Cultures de Microorganismes d'Institut Pasteur, Paris, under no. I-168.

This virus was isolated by means of the trachea swab method from broilers, which showed at an age of 4.5 weeks respiratory symptoms after previous vaccination at the age of one day with IB vaccine of the H120 type, whereby the animals found themselves at the moment of isolation in the district, which was indicated in the hereinbefore used internal notation (Gelderland).

There was found that by attenuation to SPF-chicken embryos, the isolated virus strain lost its pathogenicity for SPF chickens in a major degree, in spite of the fact that its immunising ability still remained present.

The virus strain with internal notation IBV Gelderland.901 (G.901) and deposited under no. CNCTC AO 17/81 and no. I-168, showed these beforementioned characteristics after about 13, 45 and 90 SPF type I chicken embryo passages.

There was surprisingly found during a comparing test, using conventional H120 or H52 vaccines, that the protection against IB viruses of the Massachusetts type
- measures as the amount of virus neutralizing antibodies -
had not been diminished in a significant degree, if a H-type vaccine combined with the novel IBV isolate Gelderland.901 was administered instead of the H-type vaccine alone. During this experimentation one started from intranasal application of the concerning vaccines.

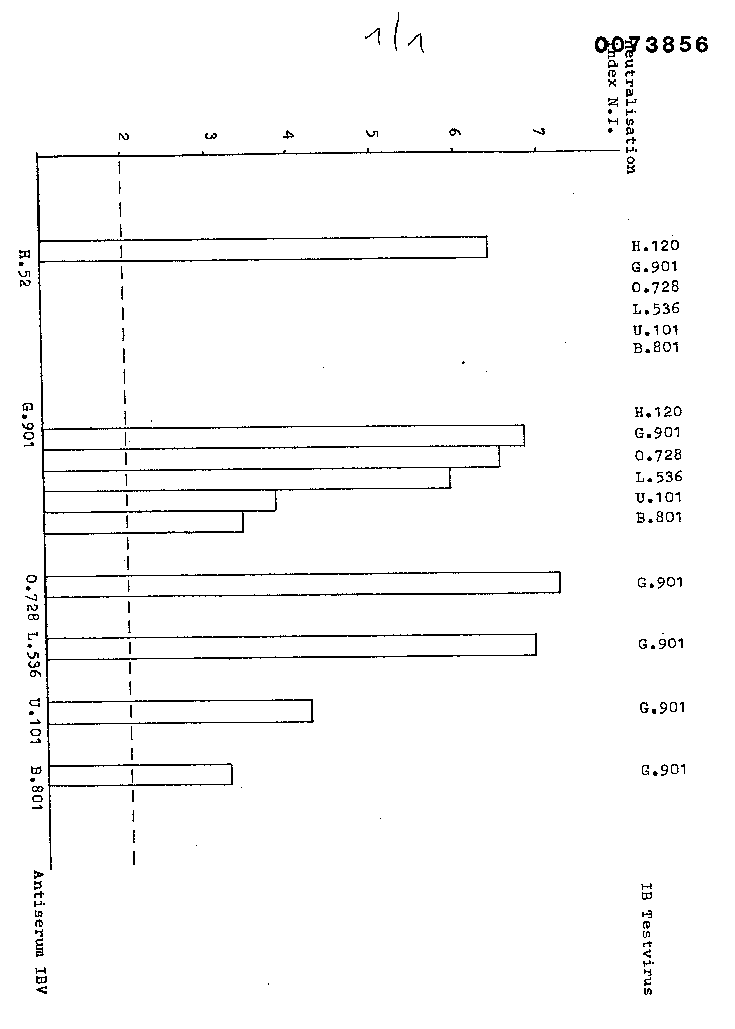

Cross neutralization tests in SPF chicken embryos and cross infection tests on SPF chickens were carried out, giving results as showed in the here-under following tables and in figure 1. A neutralization index $>$ 2.0 means that the chickens involved are protected against the specific IBV infection.

## TABLE 1

Cross neutralization test in SPF chicken embryos.

| Infectious bronchitis testvirus | Neutralization index (N.I.) with antiserum against infectious bronchitis virus | | | | |
|---|---|---|---|---|---|
| | H.52 | G.901 | O.728 | L.536 | U.101 |
| H.120 | 6.4 | 1.6 | 0.3 | 0.4 | 1.9 |
| G.901 | 0.8 | 6.8 | 7.2 | 6.9 | 4.2 |
| O.728 | ND | 6.5 | ND | ND | ND |
| L.536 | ND | 4,9 | ND | ND | ND |
| U.101 | ND | 3,8 | ND | ND | ND |

Remarks: The abbrevation "ND" means "not done".

## TABLE 2

Cross infection tests with 4 weeks old SPF chickens.

| Vaccine virus | Challenge virus | Virus reisolation |
|---|---|---|
| IBV G.901 | IBV G.901 | 0/5 positive |
| | IBV VOET (Massachusetts) | 3/5 positive |
| | IBV O.728 | 0/5 positive |
| IBV H.120 | IBV G.901 | 4/5 positive |
| | IBV VOET (Massachusetts) | 0/5 positive |
| | IBV O.728 | 5/5 positive |

The vaccine virus was administered intranasally at a dose of $10^{4.0} EID_{50}$ per bird.

The virus reisolation test was carried out 5 days after challenge using tracheaswab material. The virus reisolation technique applied was described in "Specifications for the production and control of avian live virus vaccines" of the Ministry of Agriculture, Fisheries and Food of the United Kingdom Central Veterinary Laboratory of Biological Products and Standards Department, New Haw, Weybridge, Surrey KT153 NB, 2nd Edition (1977), p.12.

From the results of tables 1 and 2 (cross neutralization and cross infection tests) it appears that the applied serotype of the novel IBV G.901 differs antigenitically from the H-strain and - having in mind the results of the challenge experiments with additional virus reisolation - shows attractive immunogenic properties as to the homologeous viruses as described in the above-mentioned European Patent Application.

Field experiments showed that in sera of broilers, reproduction chickens and laying hens, antibodies were frequently occurring against the virustype Gelderland.901.

In figure 1 the neutralization indices (vertical ax) determined for the IBV testviruses H.120, Gelderland.901, Overijssel.728, Limburg.536, Utrecht.101 and Brabant.801 respectively and for the homologeous testvirus Gelderland.901, are plotted for the six IBV antisera types H.52, Gelderland.-901, Overijssel.728, Limburg.536, Utrecht.101 and Brabant.801 on the horizontal axis.

It will be appreciated therefore that the novel IB virus is differing antigenitically in a significant degree from the usually applied H-virus and has common antigenic properties with the viruses of the beforementioned European application.

The novel isolated virus strain could be characterized by the following tests:

Chloroform treatment according to Mayr, A. et al, Virologische Arbeitsmethoden, G. Fischer Verlag, Jena, 1977, p. 285, of infectious amnion allantoic fluid obtained by cultivation of original virus containing samples from infected homogenized organ and trachea swab material in the allantoic cavity of 10 days preincubated SPF chicken eggs, resulted, in comparison with the non treated material, in a reduction of virus content from $10^{7.5}$ to $10^{1.5}$ $EID_{50}$. This experience may point to the presence of a virus agent, which is containing in his envelope a lipide, which is necessary for the infectivity.

The infectious amnion-allantoic fluid caused no agglutination with erythrocytes derived from SPF chickens.

Addition of 5-fluordesoxyuridine (FUDR) to the culture medium of chicken kidney cell cultures, serving for replication of the agent, did not influence the intracellular synthesis of the virus agent in a significant degree.

The $EID_{50}$ content of the cell material and culture medium appeared to reside on comparable levels 2,4 and 7 days after the inoculation of the virus agent, i.e. the nucleic acid to be replicated belonging to the group of the ribonucleic acid.

Examination with electron microscope showed, that the virus agent, present in the amnion allantoic fluid harvested within 30 hours after the artificial infection, possessed a diameter of about 100 nm. About 15 nm long projections were present on the surface of this virus. The virus has the size and shape of a corona virus, to which also the avian bronchitis viruses are regarded to belong.

It will be appeciated that the properties of the novel serotype of the IB-virus of the present invention make the novel virus strain especially suitable for the preparation of as well inactivated as live poultry vaccines on behalf of a more efficient protection against infectious bronchitis, especially in areas or countries, wherein the described deviating serotype according to the present application and the above-mentioned European patent application occur besides the IB viruses of the so called H-type.

More particularly virus strains of the serotype of the hereinbefore mentioned novel virus strain may successfully be applied for the preparation of mixed live and inactivated vaccines, derived as well from the novel IB strain as from the H-strain.

The novel IBV vaccines of the present invention may be obtained by propagation of the novel virus strain by methods known in the art in principle and optionally followed by inactivation by methods known in the art in principle.

For instance the virus may be propagated in embryonated SPF chicken eggs or in suitable cell cultures such as chicken kidney cell cultures. However with such a process there has to be checked whether the antigenic properties and the degree of virus replication do not significantly change.

Hereafter the cultivated virus material is collected and purified. At last one or more stabilizers and, if desired, antibiotics, such as sodium penicillin G, streptomycin or natamycin, may be added and the mixture is lyophilized.

More particularly, the seed virus concerned is inoculated under sterile conditions into the allantoic cavity of 10-11 days preincubated SPF type I chicken eggs.

After incubation for 28 to 48 hours at 37°C, the amnion-allantoic fluid of the then still living and of the specifically died (i.e. between 24 hours after the seed virus inoculation and the end of the incubation period) embryos is harvested, purified and lyophilized after optional addition of stabilizers and/or antibiotics.

According to this process, single vaccines could be prepared, containing the virus in an amount of at least $10^{4.0}$ $EID_{50}$ per dose after lyophilisation, while e.g. so prepared combined vaccines of the novel virus strain and a known H-strain and/or more other IBV-strains showed a virus content of $\geqslant 2 \times 10^{4.0}$ $EID_{50}$ per dose and preferably a content of each of the virus components of at least $10^{4.0}$ $EID_{50}$ per dose.

It will be appreciated that the present invention is also relating to novel, as well inactivated as live, IBV vaccines, which have been at least derived from the novel IB virus strain G.901 and to the application of such vaccines.

Preferably live vaccines, derived from the novel virus G.901 alone or from the H-type virus with the novel 1B-virus, are applied.

More preferably live vaccines, derived from H120 or H52 virus strain and from the G.901 virus strain are applied. The vaccines may also be applied to young chickens.

The novel life virus vaccines may be administered by the so called eyedrop- or nosedrop-, the drinking-water- or spraymethod. Vaccination by means of the novel live vaccines, according to the present invention has preferably to be carried out with poultry of an age of 1 day to 18 weeks. The novel inactivated vaccines are administered to the birds subcutaneously or intramuscularly and used for revaccination purposes only.

It will be appreciated that also combined live or inactivated vaccines, derived from the novel IB virus type G.901 and one or more completely other virus types, such as e.g. the Newcastle disease virus, adeno-like virus, infectious bursitus-virus or reo virus, are a feature of the present invention too.

For the preparation of inactivated IBV vaccines according to the present invention there may be started from e.g. an amnion-allantoic fluid, to which a suitable carrier is added, after inactivation by methods known in the art, e.g. by means of beta-propiolactone or formaline.

Preferably the virus suspension with a suitable virus content is processed to a water in oil emulsion vaccine, derived from a mineral or plant oil and one or more emulsifiers, such as non-ionic surface-active compounds derived from alkylene oxide and/or hexahydric alcohols and/or higher natural fatty acids ($C_{10}$-$C_{20}$) such as esters or ester-ethers.

Examples of the lastmentioned emulsifiers are mannide monooleate (Span® 80, Arlacel 80®, Arlacel A®) and polyoxyethylene (20) sorbitan monooleate (e.g. Tween 80®).

The volume ratio between the aqueous phase (virus fluid) and the oily phase may vary from 3:7 to 1:1 and lies preferably at a ratio of about 7:13.

The invention is illustrated by the following examples, however, without any restriction of the object of the invention to these specific embodiments.

## Example 1

**Preparation of live IB virus vaccine of the strain G.901.**

A. Cultivation of virus.

Type I SPF chicken egg, preincubated for 10 to 11 days, are inoculated into the allantoic cavity with $10^{3.0}$ to $10^{4.0}$ $EID_{50}$ IBV G.901 seed virus (0.2 ml per egg).

The eggs are candled for the first time 20 to 24 hours after the virus inoculation and all aspecificly died embryos are removed.

After an incubation period of in total 28 hours at +37°C, the amnion-allantoic fluid (AAF) is harvested.

B. Treatment of virus suspension.

After purification of the AAF by means of centrifugation for 20 minutes at 2000 r.p.m. in a cooling centrifuge and/or by filtration, 5 x $10^5$ units of sodium penicillin G and 800 mg of streptomycin per liter are added to this AAF.

The virus material is subsequently stabilized by addition of at least 3% by weight of albumin and/or mannitol.

The stabilized bulk virus material is frozen to at least -35°C and stored at such temperature until the further processing phase.

Samples of this material are meanwhile tested on their virus content by means of the $EID_{50}$ (Egg Infectious Dose 50%) assay method.

After the test results have become available, the virus material is thawn up again and filled out into lyophilization flasks.

The virus content (volume) is adjusted hereby in such a way, that at the end of the subsequent lyophilization there are still at least $10^{4.0}$ $EID_{50}$ of the concerning virus per dose present in the vaccine.

The flasks are sealed under vacuum at the end of the lyophilization process.

With the preparation of the multivalent (combined) vaccine care has to be taken that the minimal virus contents for all virus components are reached.

Example 2

Preparation of a combined live IB virus vaccine of the strain
H.52 and G.901.

A. Cultivation of virus.

Type I SPF chicken eggs, which have been preincubated for 10 to 11 days, are inoculated into the allantoic cavity with $10^{3.0}$ to $10^{4,0}$ $EID_{50}$ of H.52 or G.901 seed virus (0,2 ml in total per egg).

The eggs are candled for the first time 20 to 24 hours after the virus inoculation, and all aspecifically died embryos are removed. After an incubation period of in total 32 hours at 37°C, the AAF is harvested.

B. Treatment of the virus suspension.

After purification of the AAF by means of centrifugation for 20 minutes at 2000 r.p.m. in a cooling centrifuge and/or by filtration, 8 x $10^5$ units of sodium penicillin G and 1000 mg of streptomycine per liter are added to this AAF.

The virus material is subsequently stabilized by addition of at least 3% by weight of albumine and/or mannitol.

The stabilized bulk virus material is subsequently frozen to at least -35°C and kept at this temperature until further processing.

Meanwhile samples of this material are tested on their virus content by means of the $EID_{50}$ assay method.

The virus material is thawn up again and filled out into lyophilisation flasks after the test results have become available.

The virus content (volume) is adjusted hereby in such a way that at the end of the lyophilisation process the vaccine contains per dose at least $10^{4.0}$ $EID_{50}$ of each virus concerned. The flasks are sealed under vacuo at the end of the lyophilisation process.

During the preparation of the multivalent (combined) vaccine care has to be taken that the minimum virus content for all virus components is reached.

## Example 3

Preparation of inactivated combined IB-virus vaccine of the strains H52 and G.901.

In a similar way as described in Example 2.A, the virus was cultivated in SPF eggs and the obtained virus suspension was treated in a similar way as in Example 2.B, until the frozen phase is reached, however, without addition of antibiotics and stabilizers.

The frozen AAF is thawn and inactivated in a water bath by 0.1% of bêta-propiolactone during a period of 90 minutes at 37°C.

Consequently the virus suspension is kept overnight at +4°C. The inactivation is checked by inoculation of preincubated, embryonated SPF chicken eggs with the inactivated virus material and subsequent incubation.

The inactivated AAF is diluted if necessary, with PBS +0,3% of formaline, dependent on the virus content of each virus type, determined in the non-inactivated AAF (at least $10^{7.0}$ EID$_{50}$ per ml for all virus strains).

To the virus suspension of the four strains 3.5% of Tween 80 are added.

The inactivated virus suspension is mixed with an oil phase in the ratio of 6.5 parts of oil to 3.5 parts of virus fluid and emulsified, in a way that the average particle size of the aqueous phase is about 0.5 $\mu$.

The emulsification is carried out by means of an Ultra Turrax homogeniser or by means of passing the starting mixture through a colloid mill.

The applied oil phase has the following composition:

Marcol 5 2® (white paraffinic Esso oil)                    93,5 %

Arlacel A®, Arlacel 80® or Span 80®

(mannide monooleate)                                        6,5%.

The components of the oil phase are separately heated to 110°C in an autoclave or the mixture is sterilized by filtration.

Claims

1. Infectious bronchitis vaccines for poultry, characterized in that they are derived from at least one virus strain of a novel serotype of the infectious bronchitis virus, indicated by the internal notation Gelderland.901, which has been deposited with the Czechoslovak National Collection of Type Cultures of the Institute of Hygiene and Epidemiology in Praque under no.  CNCTC AO 17/81 and with the Collection Nationale de Cultures de Microorganismes d'Institut Pateur in Paris under no. I-168.

2. Combined infectious bronchitis vaccines, according to claim 1, characterized in that these vaccines in addition are derived from the IBV H120 or the IBV H52 of the Massachusetts type.

3. Infectious bronchitis vaccines, according to claim 2, characterized in that they are derived in addition from the IBV H52 of the Massachusetts type.

4. Live infectious bronchitis vaccine, according to claim 1-3, characterized in that they show after lyophilization a virus content of at least $10^{4.0}$ $EID_{50}$ of each of the virus strains per dose.

5. Live infectious bronchitis vaccine, according to claim 4, characterized in that they show after lyophilization a total virus content of at least $10^{4.0}$ $EID_{50}$ of each of the virus strains per dose.

6. Inactivated infectious bronchitis vaccines, according to claims 1-3, characterized in that they contain an oily phase, containing at least a mineral or plant oil and one or more suitable emulsifying agents in the form of a non-ionogenic surface active agent, derived from alkylene oxide and/or hexahydric alcohols and/or higher natural fatty acids of 10-20 carbon atoms, such as esters or ester-ethers as essential ingredients.

7. Infectious bronchitis virus strains of a novel serotype indicated by means of the internal notation Gelderland.901, deposited at the Czechoslovak National Collection of Type Cultures of the Institute of Hygiene and Epidemiology in Prague under no. CNCTC AO 17/81 and deposited at the Collection Nationale de Cultures de Microorganismes d'Institut Pasteur, Paris, under no. I-168.

8. Process for the preparation of infectious bronchitis vaccines for poultry according to claim 1, characterized in that at least one of the virus strains is propagated as seed virus in fertilized chicken eggs or in suitable cell cultures such as SPF chicken kidney cell cultures, whereafter the cultivated virus material is collected and purified, followed by lyophilization of the mixture after the optional addition of stabilizers and/or antibiotics.

9. Process according to claim 8, characterized in that the seed virus is inoculated under sterile conditions in the allantoic cavity of embryonated type I SPF chicken eggs, which have been preincubated for 10-11 days, whereafter the amnion-allantoic fluid of still living and specifically died embryos is harvested after incubation at +37°C during 28 to 48 hours, purified and after addition of stabilizers and optionally antibiotics, lyophilized.

10. Process according to claims 8 and 9, characterized in that single vaccines are prepared, which are containing after lyophilization at least $10^4.0$ $EID_{50}$ per dose of the concerning virus.

11. Process for the preparation of combined vaccines according to claims 2 and 3, characterized in that the virus suspension, obtained by cultivation of at least one virus strain of the novel serotype of the infectious bronchitis virus indicated by the internal notation Gelderland.901 and subsequent purification and mixing, after optional addition of stabilizers and/or antibiotics, with a virus suspension, obtained by cultivation of an IBV H120 or an IBV H52, purification and optional addition of stabilizers and antibiotics, followed by lyophilization.

12. Process for the preparation of combined vaccines according to claim 11, characterized in that vaccines are prepared, which are containing after lyophilization a virus content being at least $10^4.0$ $EID_{50}$ of each of the virus components per dose.

13. Process for preventing poultry against infectious bronchitis by vaccinating it according to methods known per se, with the vaccines according to claims 1-6.

14. Inactivated infectious bronchitis vaccines, according to claim 6, characterized in that they are derived from besides one or more IBV strains in addition from one or more totally different virus types such as Newcastle disease virus, adenolike virus, infectious bursitus virus and/or reo virus.

Neutralisation
Index N.I.

IB Testvirus

H.120
G.901
0.728
L.536
U.101
B.801

H.120
G.901
0.728
L.536
U.101
B.801

G.901

G.901

G.901

G.901

7

6

5

4

3

2

H.52    G.901    0.728 L.536  U.101   B.801    Antiserum IBV

European Patent Office

**PARTIAL EUROPEAN SEARCH REPORT**
which under Rule 45 of the European Patent Convention
shall be considered, for the purposes of subsequent
proceedings, as the European search report

0073856

EP 81 20 0960

## DOCUMENTS CONSIDERED TO BE RELEVANT

| Category | Citation of document with indication, where appropriate, of relevant passages | Relevant to claim |
|---|---|---|
| D/A | <u>EP - A - 0 030 063</u> (GIST-BROCADES)<br>.<br>* the entire document * | 1-12,<br>14 |
| | -- | |
| A | BIOLOGICAL ABSTRACTS, vol. 72,<br>April 1981,<br>abstract no. 24308<br>PHILADELPHIA, Pa. (US)<br>& Vet. Rec. 108 (4) 72-75, 1981<br>P.J. WYETH et al.: "Immune respon-<br>ses of breeding chickens to tri-<br>valent oil emulsion vaccines.<br>Responses to Newcastle disease<br>and infections bursa disease"<br><br>* the entire abstract * | 6,14 |
| | -- | |
| A | BIOLOGICAL ABSTRACTS, vol. 63,<br>June 1977<br>abstract no. 63802<br>PHILADELPHIA, Pa. (US)<br>& Ann. Med. Vet. 120(3), 199-204<br>1976<br>G. MEULEMANS et al.: "Isolation<br>of a new serotype of the avian<br>infectious bronchitis virus in | ./. |

**CLASSIFICATION OF THE APPLICATION (Int. Cl.³)**

C 12 N    7/00
A 61 K 39/215
              39/295

**TECHNICAL FIELDS SEARCHED (Int. Cl.³)**

A 61 K

## INCOMPLETE SEARCH

The Search Division considers that the present European patent application does not comply with
the provisions of the European Patent Convention to such an extent that it is not possible to carry
out a meaningful search into the state of the art on the basis of some of the claims.
Claims searched completely:     1-12,14
Claims searched incompletely:
Claims not searched:      13 (Method for treatment of
Reason for the limitation of the search:    the human or animal body
by surgery or therapy (see article 52(4)
of the European Patent Convention)

**CATEGORY OF CITED DOCUMENTS**

X: particularly relevant if taken alone
Y: particularly relevant if combined with another document of the same category
A: technological báckground
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| The Hague | 15-04-1982 | MARIE |

EPO Form 1505.1   06.78

European Patent Office

**PARTIAL EUROPEAN SEARCH REPORT**

| | DOCUMENTS CONSIDERED TO BE RELEVANT | | CLASSIFICATION OF THE APPLICATION (Int Cl ³) |
|---|---|---|---|
| Category | Citation of document with indication, where appropriate, of relevant passages | Relevant to claim | |
| | Belgium" | | |
| | * the entire abstract * | 7 | |
| | --- | | |
| A | BIOLOGICAL ABSTRACTS, vol. 62, August 15, 1976 abstract no. 20905 PHILADELPHIA, Pa. (US) & Avian Dis 20 (1), 199-201, 1976 J.K. ROSENBERGER et al.: "Cross-protection studies with a Holland strain (Noblis H-52) of infectious bronchitis virus" | | **TECHNICAL FIELDS SEARCHED (Int Cl.³)** |
| | * the entire abstract * | 1,6,7 | |
| | --- | | |
| A | BIOLOGICAL ABSTRACTS, vol. 62, July 15, 1976 abstract no. 8254 PHILADELPHIA, Pa (US) & Avian Dis 20(1), 42-48, 1976 R.W. WINTERFIELD et al.: "Immunity to infectious bronchitis virus from spray vaccination with derivatives of a Holland strain" | | |
| | * entire abstract * | 1,6,7 | |

EPO Form 1505.3 06.78